(19) Europäisches Patentamt — European Patent Office — Office européen des brevets

(11) **EP 4 079 157 B1**

(12) **EUROPEAN PATENT SPECIFICATION**

(45) Date of publication and mention
of the grant of the patent:
**31.12.2025 Bulletin 2026/01**

(21) Application number: **21738164.9**

(22) Date of filing: **05.01.2021**

(51) International Patent Classification (IPC):
*A01N 31/04* (2006.01)  *A61L 2/20* (2006.01)
*A01P 3/00* (2006.01)  *A61L 9/00* (2006.01)
*A61L 9/01* (2006.01)  *A61L 9/14* (2006.01)
*A01N 25/34* (2006.01)  *A01P 1/00* (2006.01)
*A01N 25/06* (2006.01)  *A01N 31/06* (2006.01)

(52) Cooperative Patent Classification (CPC):
(C-Sets available)
**A01P 3/00; A01N 25/06; A01N 25/34; A01N 31/04;
A01N 31/06; A01P 1/00; A61L 9/14;** A61L 2209/21
(Cont.)

(86) International application number:
**PCT/JP2021/000112**

(87) International publication number:
**WO 2021/141027 (15.07.2021 Gazette 2021/28)**

(54) **INDOOR-USE SPACE STERILIZING AGENT AND INDOOR-USE SPACE STERILIZING APPARATUS**

STERILISATIONSVORRICHTUNG FÜR INNENRAUM VERWENDUNG

AGENT DE STÉRILISATION D'ESPACE À USAGE INTÉRIEUR ET APPAREIL DE STÉRILISATION D'ESPACE À USAGE INTÉRIEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.01.2020 JP 2020000253**

(43) Date of publication of application:
**26.10.2022 Bulletin 2022/43**

(73) Proprietor: **FUMAKILLA LIMITED
Tokyo 101-8606 (JP)**

(72) Inventor: **MURAKAMI, Kanako
Hatsukaichi-shi, Hiroshima 739-0494 (JP)**

(74) Representative: **Germain Maureau
12, rue Boileau
69006 Lyon (FR)**

(56) References cited:
**EP-B1- 1 183 053        WO-A1-2011/125728
WO-A1-2012/043437      CN-A- 1 458 471
JP-A- 2001 072 503      JP-A- 2003 504 121
JP-A- 2003 504 121      JP-A- 2022 102 449
US-A- 4 806 526        US-A1- 2010 189 599
US-A1- 2010 189 599**

**(Cont. next page)**

(52) Cooperative Patent Classification (CPC): (Cont.)

C-Sets
**A01N 25/34, A01N 31/04;**
**A01N 31/06, A01N 25/06**

**Description**

TECHNICAL FIELD

The present invention relates to an indoor space sanitizing agent and an indoor space sanitizing device used, for example, in an indoor space. The here disclosed indoor space sanitizing device comprises benzyl alcohol as an active component;a holding body having air permeability and configured to hold the indoor space sanitizing agent, wherein the indoor space sanitizing agent held by the holding body is spread by natural convection of air, and a cartridge including the holding body.

BACKGROUND ART

Chlorine dioxide gas has been used as an agent for sanitizing a space (see, e.g., Patent Document 1). In Patent Document 1, the chlorine dioxide gas is generated by chemical reaction between an aqueous solution of stabilized chlorine dioxide and citric acid, and the generated chlorine dioxide gas is gradually released into the air.

Further, a fungicide using benzyl alcohol as a volatile fungicide and further containing an antioxidant, an odor reducing agent, and a volatilization adjusting agent has been known (see, e.g., Patent Document 2).

CITATION LIST

PATENT DOCUMENT

Patent Document 1: Japanese Unexamined Patent Publication No. 2014-014589
Patent Document 2: Japanese Unexamined Patent Publication No. 2001-072503

SUMMARY OF THE INVENTION

TECHNICAL PROBLEM

The chlorine dioxide gas is preferably generated by the chemical reaction upon use as in Patent Document 1, because the chlorine dioxide gas is unstable. However, in this method, it is practically difficult to continuously release a stable concentration of the gas for a long period of time. Moreover, there has been a safety concern about chlorine dioxide upon use in a high concentration. For this reason, there has been a demand for an agent capable of stably sanitizing a space in a safe concentration of the agent.

Patent Document 2 discloses that benzyl alcohol has a volatile antifungal effect. However, Patent Document 2 merely discloses in an embodiment an antifungal effect (an effect on fungi) in a closed space of about 500 ml. An effect in an everyday space is not clear; in particular, an effect on bacteria which might cause a problem in an everyday space is totally unclear.

EP1183053 describes a composition for the sanitization of a room, wherein the composition comprises benzyl alcohol. An air stream helps to provide an aerosol for air treatment. JP2001072503 describes the use of benzyl alcohol as a disinfecting anti mildew agent. The composition comprises an odour lowering agent and an agent that should protect benzyl alcohol from being oxidized. A vaporization regulator contains a porous carrier. A porous substrate should be present as carrier to develop the slow release of the adsorbed anti-fungal agent.

The present invention has been made in view of the foregoing, and it is an object of the present invention to obtain a high safety sanitizing effect that is stable over a long period of time.

SOLUTION TO THE PROBLEM

For accomplishing the above-described object, an indoor space sanitizing agent contains benzyl alcohol as an active component.

According to this configuration, benzyl alcohol volatilizes to be diffused in an indoor space, thereby sanitizing the space. Since benzyl alcohol is chemically stable and has a moderate level of evaporation properties, benzyl alcohol can be evaporated at a stable concentration in the indoor space, and a stable sanitizing effect can be produced over a long period of time. Benzyl alcohol is safer than chlorine dioxide gas. Further, benzyl alcohol has an antibacterial effect, and produces an effect as an antibacterial agent. The term "indoor" indicates a place other than the outside of a room (an open space), and for example, includes living spaces such as the inside of a house, an office, a factory, and a store. In addition, the "indoor" also includes individual spaces such as a lavatory and a bathroom and housing spaces such as the inside of a warehouse, a closet, a shoebox, and a refrigerator.

A second aspect is directed to an indoor space sanitizing device including an indoor space sanitizing agent containing benzyl alcohol as an active component and a holding body configured to hold the indoor space sanitizing agent.

According to this configuration, the indoor space sanitizing agent is released gradually from the holding body, and therefore, the sanitizing effect lasts over a long period of time.

This can be adopted also as a space deodorizing method in which benzyl alcohol as a deodorizing active component is released into a space.

According to this configuration, benzyl alcohol as the deodorizing active component is released into the space, thereby making it possible to eliminate various types of malodors in a house. Various malodorous components which can be eliminated by benzyl alcohol may include, for example, ammonia, isovaleric acid, diacetyl, trimethylamine, mercaptan, trichloroanisole, and nonenal.

Benzyl alcohol is a compound which naturally exists as a precursor of an aroma such as jasmine and is also used as a food additive and highly safe. Further, benzyl alcohol is stable as a compound; therefore, benzyl alcohol can be released into the air at a stable concentration.

According to this configuration, benzyl alcohol does not need to be brought into direct contact with a source of the malodor, and may only be released into the space. Thus, the entirety of the space can be deodorized even when the source of the malodor is not recognized.

Benzyl alcohol is sprayed into the space from an aerosol container storing the benzyl alcohol together with a propellant. According to this configuration, benzyl alcohol can be vigorously sprayed into the space, and can be spread across a wide area.

A body impregnated with benzyl alcohol has been prepared and air is sent to the impregnated body to release the benzyl alcohol into the space. According to this configuration, benzyl alcohol can be released continuously into the space over a long period of time, and the effect of benzyl alcohol can be continuously produced.

It is possible to release benzyl alcohol into the space such that the benzyl alcohol concentration in the air is 0.05 ppm or more. Thus, a deodorizing effect can be further enhanced.

The agent can be adopted also as a space virus inactivation agent, which contains benzyl alcohol as an active component for virus inactivation.

The solution to the problem may include a virus inactivation method, in which benzyl alcohol as an active component for virus inactivation is released into a space.

According to this configuration, benzyl alcohol as the active component for virus inactivation is released into the space, so that not only viruses floating in the air but also viruses adhering to an object in the space can be inactivated.

ADVANTAGES OF THE INVENTION

As described above, benzyl alcohol as a deodorizing active component is released into a space, which can not only produce sanitizing and antibacterial effects in a space, but can also eliminate various malodors at once, and can further inactivate viruses in the space.

BRIEF DESCRIPTION OF THE DRAWINGS

FIG. 1 is a perspective view of an indoor space sanitizing device according to a first embodiment of the present invention as viewed from above.

FIG. 2 is a vertical cross-sectional view of the indoor space sanitizing device.

FIG. 3 is a perspective view of a cartridge as viewed from above.

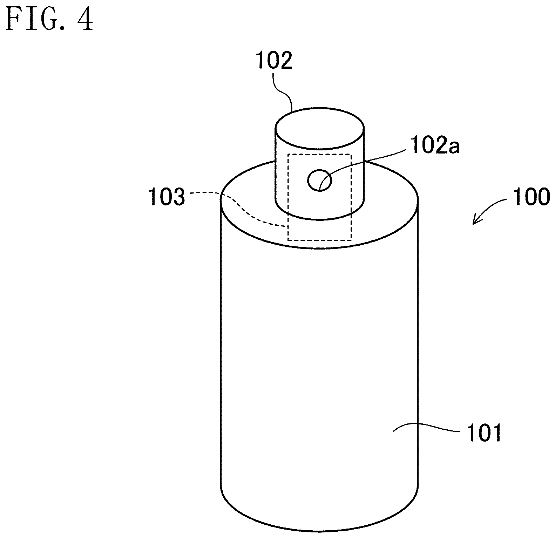

FIG. 4 is a perspective view of an aerosol product according to a second embodiment of the present invention.

FIG. 5 is a graph showing a deodorizing effect on ammonia.

FIG. 6 is a graph showing a deodorizing effect on isovaleric acid.

FIG. 7 is a graph showing a deodorizing effect on diacetyl.

FIG. 8 is a graph showing a deodorizing effect on trimethylamine.

FIG. 9 is a graph showing a deodorizing effect on mercaptan.

FIG. 10 is a graph showing a spatial contact effect on an influenza virus.

FIG. 11 is a graph showing a spatial contact effect on feline calicivirus.

FIG. 12 is a graph showing a direct contact effect on an influenza virus.

FIG. 13 is a graph showing a direct contact effect on feline calicivirus.

DESCRIPTION OF EMBODIMENT

Hereinafter, embodiments of the present invention will be described in detail with reference to the drawings. The description of preferred embodiments below is merely an example in nature, and is not intended to limit the scope, application, or use of the present invention.

(First Embodiment)

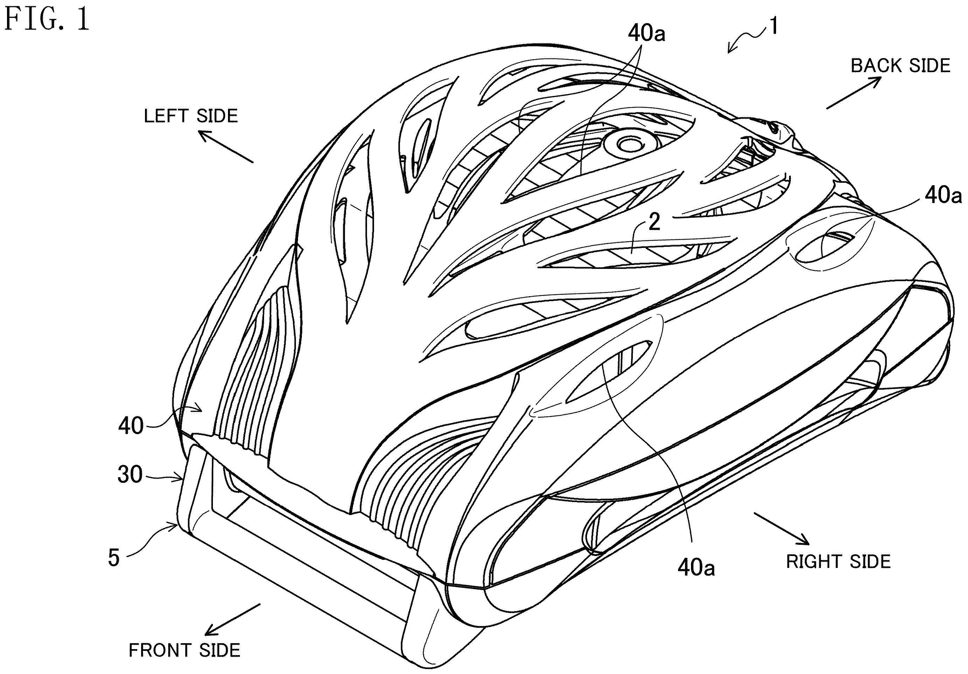

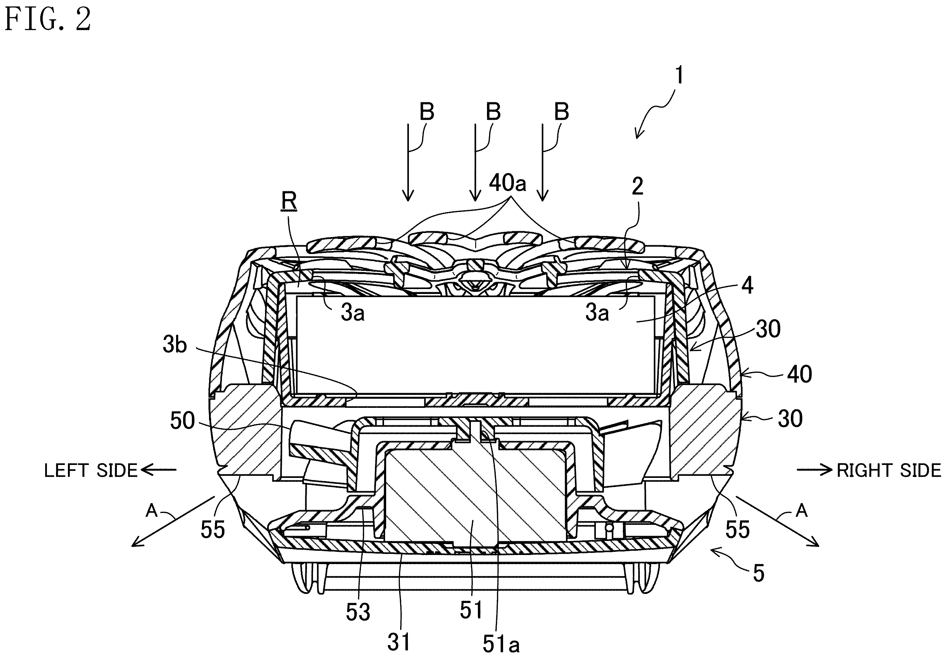

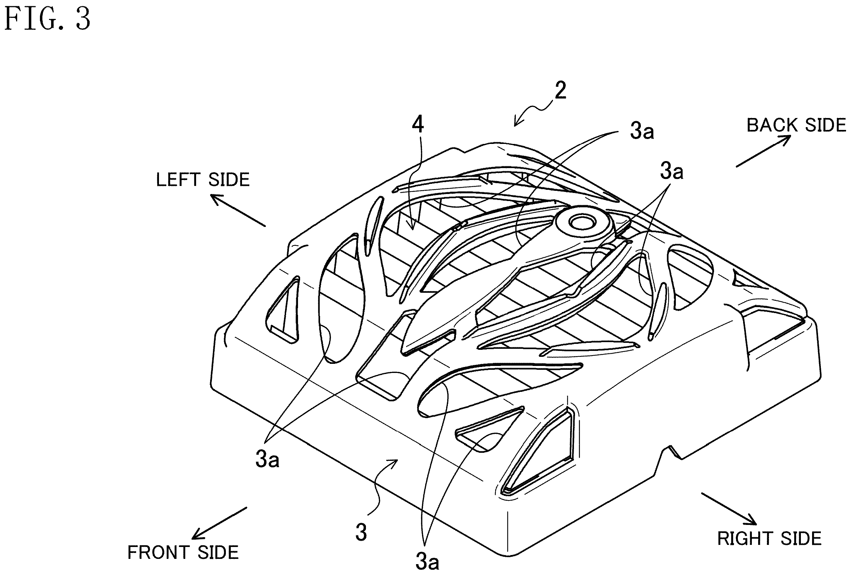

FIG. 1 is a perspective view of an indoor space sanitizing device 1 according to a first embodiment of the present invention as viewed from above. The shape and structure of the indoor space sanitizing device 1 of the present first embodiment are merely examples, and are not limited to those shown in the figure.
The indoor space sanitizing device 1 includes a cartridge 2 (shown in FIGS. 2 and 3) and a device body 5 to which the cartridge 2 is removably attached. For the sake of convenience in description of the first embodiment, one side of the indoor space sanitizing device 1 in a longitudinal direction is taken as a front side and the other side in the longitudinal direction is taken as a back side as shown in each figure. The right and left when the indoor space sanitizing device 1 is viewed from the front side are taken as right and left sides.
First, as shown in FIGS. 2 and 3, the configuration of the cartridge 2 will be described. The cartridge 2 includes a case 3 and a liquid agent holding body (impregnated body) 4 holding a liquid agent (an indoor space sanitizing agent) containing benzyl alcohol as a deodorizing active component. The case 3 is a body for housing the liquid agent holding body 4. Examples of the liquid agent held in the liquid agent holding body 4 include, as a liquid agent having the property of volatilizing at room temperatures, an indoor space sanitizing agent containing benzyl alcohol as a sanitizing active component. The liquid agent holding body 4 may hold the indoor space sanitizing agent by being impregnated with the indoor space sanitizing agent or may hold the indoor space sanitizing agent by having the indoor space sanitizing agent adhered to a surface of the liquid agent holding body 4. The indoor space sanitizing agent may contain a component other than benzyl alcohol. Since benzyl alcohol produces a deodorizing effect, as will be described later, benzyl alcohol can also be called a deodorant. Moreover, since benzyl alcohol also produces an antiviral effect, as will be described later, benzyl alcohol can also be called an antivirus agent.
The liquid agent holding body 4 is made of a fabric material in a pleat shape including folds forming continuous wave shape. For example, the fabric material to be used may include, but not limited to, non-woven fabric and woven fabric, and other materials (e.g., a foamed material having air permeability) as long as the materials have air permeability and can hold the liquid agent. The liquid agent is held substantially uniformly across the entirety of the liquid agent holding body 4.
Air vent holes 3a are formed at an upper wall portion of the case 3. As shown in FIG. 2, air vent holes 3b are also formed at a lower wall portion of the case 3.

(Configuration of Device Body 5, not according to the invention)

The configuration of the device body 5 will be described with reference to FIGS. 1 and 2. The device body 5 has a base member 30, a fan 50 as an air blower, an electric motor 51 configured to drive the fan 50, and a battery (not shown) configured to supply power to the electric motor 51. The fan 50, the electric motor 51, and the battery are housed in the base member 30 of the device body 5.
The base member 30 is formed of a resin material, for example. The electric motor 51 is fixed to a substantially center portion of the base member 30. A center portion of the fan 50 is fixed so as to rotate integrally with an output shaft 51a of the electric motor 51. The fan 50 is a known fan, and when rotatably driven by the electric motor 51, sucks air from above and discharges the air from the side.
A cartridge housing space R for housing, in a replaceable manner, the cartridge 2 including the liquid agent holding body 4 is formed above the fan 50. This cartridge housing space R is a space defined by the device body 5 and a cover 40 attached to the device body 5.

As shown in FIG. 2, liquid agent release holes 55 for releasing air sent to the liquid agent holding body 4 by the fan 50 are formed on both right and left sides of the base member 30 of the device body 5. Upstream ends of the liquid agent release holes 55 communicate with a space where the fan 50 is arranged in the device body 5. Air having flowed through the liquid agent release holes 55 easily flows toward the outside of the indoor space sanitizing device 1 as indicated by arrows A.

As shown in FIG. 1, the cover 40 is, for example, formed of a resin material, and covers the cartridge housing space R from above. Air vent holes 40a are formed at the cover 40.

(Operation of Indoor Space Sanitizing Device 1)

Next, operation of the indoor space sanitizing device 1 will be described. Due to an air flow, air above the cover 40 is, as indicated by arrows B in FIG. 2, sucked into the cartridge housing space R through the air vent holes 40a of the cover 40. Since the cartridge 2 is housed in the cartridge housing space R, the sucked air flows toward the cartridge 2, and flows into the case 3 through inlet openings 3a formed at an upper portion of the case 3 of the cartridge 2. The air having flowed into the case 3 flows out of the case 3 through the air vent holes 3b formed at the lower wall portion of the case 3. The air having flowed out of the case 3 and containing the liquid agent flows into the right and left liquid agent release holes 55, 55 of the device body 5, and is thereafter released into an outer space through each liquid agent release hole 55 as indicated by the arrows A in FIG. 2.

The configuration of the indoor space sanitizing device according to the invention is limited to the configuration below. The indoor space sanitizing device is configured by only the cartridge 2. In this case, the air is not forcibly sent, but natural convection of air can release and spread the indoor space sanitizing agent, the space deodorant, and the antivirus agent into a space. Alternatively, the case 3 of the cartridge 2 may be omitted, and only the holding body holding the indoor space sanitizing agent, the space deodorant, and the antivirus agent may be used to release and spread the indoor space sanitizing agent, the space deodorant, and the antivirus agent into a space.

(Deodorizing Method)

Next, a space deodorizing method will be described. A target space may include, for example, a room of a general house, an office, a store, a classroom, various event spaces, a vehicle cabin, an airplane cabin, and a ship cabin. Spaces other than those may also be target spaces.

Specifically, in the case of using the indoor space sanitizing device 1 the indoor space sanitizing device 1 is operated to allow the benzyl alcohol, as the deodorizing active component, to be released into the space. The amount of release of the space deodorant is adjustable by the amount of the space deodorant held by the liquid agent holding body 4.

It is preferable to close a door or a window of a room before release of the space deodorant into the space, but the door or the window may be closed immediately after the release of the space deodorant into the space. It is also preferable to ventilate the space after a lapse of a predetermined time after the release of the space deodorant into the space, but the ventilation is not needed if the benzyl alcohol concentration in the air is extremely low. It is possible to set the amount of release of the space deodorant and set the amount of release of the space deodorant per unit time according to the size of the space to be treated. With this configuration, the benzyl alcohol concentration in the air in the space to be treated is adjustable within a proper range.

The upper limit of the benzyl alcohol concentration in the air in the space to be treated is set in consideration of the influence on a human body. In the case of a formulation for constant release chronic toxicity needs to be taken into consideration. In this case, the amount of release of the benzyl alcohol is set such that the benzyl alcohol concentration in the air is 5.565 ppm or less, preferably 5.00 ppm or less, in a preferred embodiment.

The amount of release of the benzyl alcohol is set such that the benzyl alcohol concentration in the air is 0.05 ppm or more in a preferred embodiment. With this configuration, various types of malodors in a house can be deodorized. Various malodorous components in a house may include, for example, ammonia, isovaleric acid, diacetyl, trimethylamine, mercaptan, trichloroanisole, nonenal, etc. The space deodorant and the space deodorizing method using the space deodorant according to the present embodiment produce a high deodorizing effect on ammonia, isovaleric acid, diacetyl, trimethylamine, mercaptan, trichloroanisole, and nonenal. The present invention can also be called a malodorous component reducing agent and a malodorous component reducing method for removing or reducing a malodorous component contained in the air.

[Example]

Next, an example of the present invention will be described. The present invention is not limited to the example.

(Deodorizing Effect Confirmation Test for Ammonia)

In this test, a space deodorant containing benzyl alcohol as an active component was volatilized into a space, and a deodorizing effect on a malodorous component in the space was checked. That is, the efficacy was checked not in a state in which benzyl alcohol is liquid, but in a state in which benzyl alcohol in the form of vapor is released into the space.

First, a deodorizing effect confirmation test for the deodorizing effect of the space deodorant on ammonia as one of various types of malodors in a house will be described.

1. Employ a glass cylinder with a volume of 10 L, close an upper end of the glass cylinder with a glass lid, and close a lower end as well in a similar manner.

2. Put a paper filter (ADVANTEC, No5A, 90 mm) in a petri dish and place the petri dish in the glass cylinder. Apply ammonia to the paper filter. As the ammonia, 0.4% ammonia water was provided, and 0.25 mL of this ammonia water was applied.

3. Wait until the inside of the glass cylinder is filled with ammonia, and thereafter, take the paper filter out of the glass cylinder.

4. Apply a predetermined amount of a test agent to another paper filter, and place the paper filter in the glass cylinder. The test agent was a space deodorant (benzyl alcohol). A benzyl alcohol of 0.0022 mg was applied to the paper filter. After volatilization of the whole amount of the benzyl alcohol, the benzyl alcohol concentration in the air in the glass cylinder was 0.05 ppm.

5. Measure time elapsed from immediately after the paper filter, to which the test agent had been applied, was placed in the glass cylinder and the glass cylinder was closed with the lid, and measure the concentration of a malodorous component in the air in the glass cylinder by means of a detector tube in a lapse of each predetermined time. The detector tube was "GASTEC Gas Detector Tube No. 36L Ammonia."

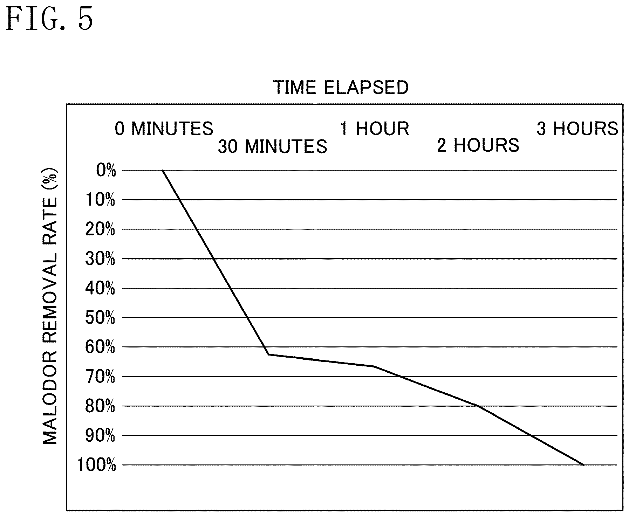

Measurement results are shown in a graph of FIG. 5. The horizontal axis of the graph shown in FIG. 5 represents the time elapsed, and the vertical axis represents a malodor removal rate (the rate of malodor removal by the test agent) as compared to Blank. The Blank indicates a case where the paper filter to which the test agent had been applied was not placed in the glass cylinder in Step 4. The malodor removal rate A was calculated by the following formula.

A = (Measurement Value of Blank - Measurement Value When Test Agent was Used)/Measurement Value of Blank $\times$ 100 (%)

As shown in the graph, the rate of malodor removal by the test agent was 63% when 30 minutes elapsed, 67% when one hour elapsed, 80% when two hours elapsed, and 100% when three hours elapsed, and substantially all the ammonia was removed from inside the glass cylinder when three hours elapsed after treatment for the space in the glass cylinder. According to the determination criteria set by Air Fresheners and Deodorizers Conference, it is determined that there is a deodorizing effect if 90% of a malodorous component is removed within 10 hours in a similar test (using an air bag instead of the glass cylinder). Since 100% was removed when three hours elapsed in this test, it can be said according to the above-described determination criteria that there was the deodorizing effect on ammonia.

(Deodorizing Effect Confirmation Test for Isovaleric Acid)

Next, a deodorizing effect confirmation test for the deodorizing effect of the space deodorant on isovaleric acid as one of various types of malodors in a house will be described. A test system is similar to that in the test for ammonia. A difference is that isovaleric acid was applied to the paper filter in Step 2. Isovaleric acid as a 0.4% aqueous solution was provided, and 0.3 mL of the aqueous solution was applied. The detector tube used for the concentration measurement in Step 5 was "GASTEC Gas Detector Tube No. 81L Acetic Acid."

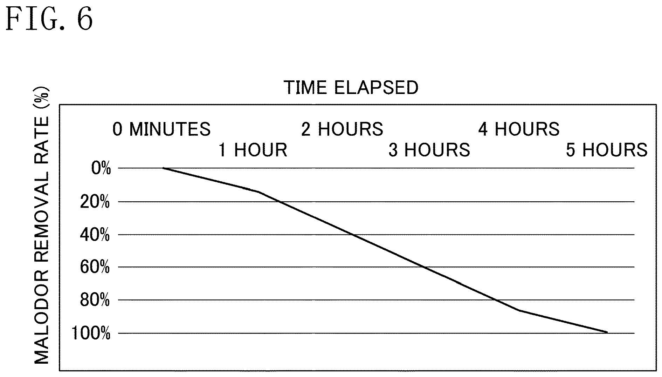

Measurement results are shown in a graph of FIG. 6. The horizontal axis of the graph shown in FIG. 6 represents the time elapsed, and the vertical axis represents a malodor removal rate (the rate of malodor removal by the test agent) as compared to Blank. The Blank indicates a case where the paper filter to which the test agent had been applied was not placed in the glass cylinder in Step 4. A formula for calculating the malodor removal rate is the same as that in the test for ammonia.

As shown in the graph, the rate of malodor removal by the test agent was 13% when one hour elapsed, 38% when two hours elapsed, 62% when three hours elapsed, 87% when four hours elapsed, and 100% when five hours elapsed, and substantially all the isovaleric acid was removed from the glass cylinder when five hours elapsed after treatment for the space in the glass cylinder. Thus, it can be said that there was the deodorizing effect.

(Deodorizing Effect Confirmation Test for Diacetyl)

Next, a deodorizing effect confirmation test for the deodorizing effect of the space deodorant on diacetyl as one of various types of malodors in a house will be described. A test system is similar to that in the test for ammonia. A difference is that diacetyl was applied to the paper filter in Step 2. As the diacetyl, a 0.1% diacetyl solution was diluted with ion-exchanged water, and 300 $\mu$L of this dilution was applied. The detector tube was "GASTEC Gas Detector Tube Acetaldehyde."

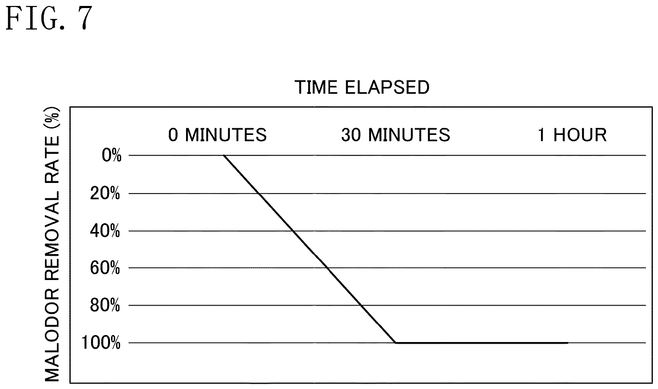

Measurement results are shown in a graph of FIG. 7. The horizontal axis of the graph shown in FIG. 7 represents the time elapsed, and the vertical axis represents a malodor removal rate (the rate of malodor removal by the test agent) as compared to Blank. The Blank indicates a case where the paper filter to which the test agent had been applied was not placed in the glass cylinder after the inside of the glass cylinder was filled with diacetyl as described above. A formula for calculating the malodor removal rate is the same as that in the test for ammonia.

As shown in the graph, the rate of malodor removal by the test agent was 50% when 30 minutes elapsed and 100% when one hour elapsed, and substantially all the diacetyl was removed from the glass cylinder when one hour elapsed after treatment for the space in the glass cylinder. Thus, it can be said that there was the deodorizing effect.

(Deodorizing Effect Confirmation Test for Trimethylamine)

Next, a deodorizing effect confirmation test for the deodorizing effect of the space deodorant on trimethylamine as one of various types of malodors in a house will be described. A test system is similar to that in the test for ammonia. A difference is that trimethylamine was applied to the paper filter in Step 2. As the trimethylamine, a 0.1% trimethylamine solution was diluted with ion-exchanged water, and a 300 $\mu$L of this dilution was applied. The detector tube used for concentration measurement in Step 5 was "GASTEC Gas Detector Tube Amines."

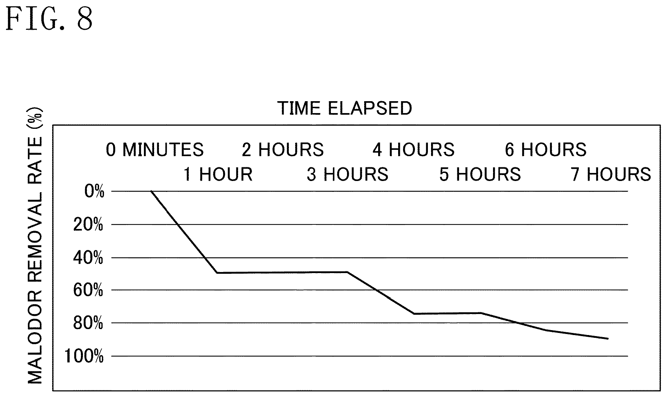

Measurement results are shown in a graph of FIG. 8. The horizontal axis of the graph shown in FIG. 8 represents the time elapsed, and the vertical axis represents a malodor removal rate (the rate of malodor removal by the test agent) as compared to Blank. The Blank indicates a case where the paper filter to which the test agent had been applied was not placed in the glass cylinder after the inside of the glass cylinder was filled with trimethylamine as described above. A formula for calculating the malodor removal rate is the same as that in the test for ammonia.

As shown in the graph, the rate of malodor removal by the test agent was 50% when one hour elapsed, when two hours elapsed, and when three hours elapsed, 75% when four hours elapsed and when five hours elapsed, 85% when six hours elapsed, and 90% when seven hours elapsed, and most of trimethylamine was removed from the glass cylinder when seven hours elapsed after treatment for the space in the glass cylinder. Thus, it can be said that there was the deodorizing effect.

(Deodorizing Effect Confirmation Test for Mercaptan)

Next, a deodorizing effect confirmation test for the deodorizing effect of the space deodorant on mercaptan as one of various types of malodors in a house will be described. A test system is similar to that in the test for ammonia. A difference is that 200 $\mu$L of a mercaptan standard solution was applied to the paper filter in Step 2. The detector tube used for concentration measurement in Step 5 was "GASTEC Gas Detector Tube Mercaptan."

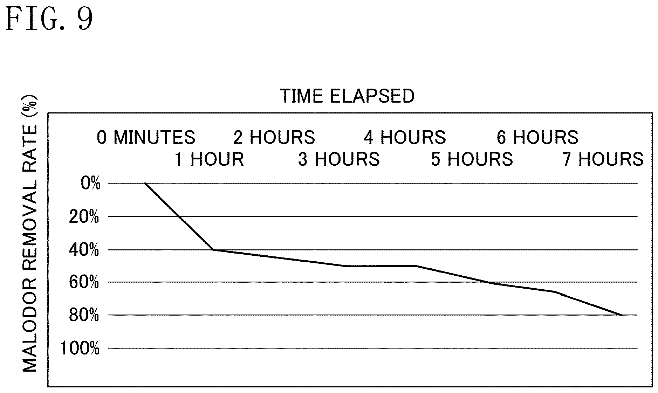

Measurement results are shown in a graph of FIG. 9. The horizontal axis of the graph shown in FIG. 9 represents the time elapsed, and the vertical axis represents a malodor removal rate (the rate of malodor removal by the test agent) as compared to Blank. The Blank indicates a case where the paper filter to which the test agent had been applied was not placed in the glass cylinder after the inside of the glass cylinder was filled with mercaptan as described above. A formula for calculating the malodor removal rate is the same as that in the test for ammonia.

As shown in the graph, the rate of malodor removal by the test agent was 40% when one hour elapsed, 44% when two hours elapsed, 50% when three hours elapsed and when four hours elapsed, 60% when five hours elapsed, 65% when six hours elapsed, and 80% when seven hours elapsed, and most of mercaptan was removed from the glass cylinder when seven hours elapsed after treatment for the space in the glass cylinder. Similar deodorizing tests for mercaptan were performed using other deodorants (a commercially available deodorant using superabsorbent polymer, and a green tea extract that is a known deodorizing component) to find that the removal rate after a lapse of seven hours was 60% or less in each of the tests. It can thus be said that benzyl alcohol produces a significant deodorizing effect on mercaptan as compared to other deodorizing components.

(Deodorizing Effect Confirmation Test for Trichloroanisole)

Next, a deodorizing effect confirmation test for the deodorizing effect of the space deodorant on trichloroanisole (2,4,6-trichloroanisole) as one of various types of malodors in a house will be described. Since a proper detector tube for measuring the concentration of trichloroanisole was not available, the deodorizing effect was checked by a sensory test.

1. Employ a glass cylinder with a volume of 10 L, close an upper end of the glass cylinder with a glass lid, and close a lower end as well in a similar manner.
2. Drop 1 mL of 4μg/ml trichloroanisole in 99 % ethanol onto a paper filter, and place the paper filter in the glass cylinder.
3. Wait until the inside of the glass cylinder is filled with trichloroanisole, and thereafter, take the paper filter out of the glass cylinder.
4. Apply a predetermined amount of a test agent to another paper filter, and place the paper filter in the glass cylinder. The test agent was a space deodorant (benzyl alcohol). A benzyl alcohol of 0.0022 mg was applied to the paper filter. After volatilization of the whole amount of the benzyl alcohol, the benzyl alcohol concentration in the air in the glass cylinder was 0.05 ppm.
5. Perform a sensory check of the smell in the glass cylinder when one hour elapsed from immediately after the paper filter, to which the test agent had been applied, was placed in the glass cylinder and the glass cylinder was closed with the lid. The concentration of trichloroanisole was set with reference to a TCA concentration in a sensory panel off-flavor discrimination ability measurement method by Food Off-Flavor Study Group of Tokyo Kasei University.

Blank was also provided. The Blank indicates a case where the paper filter to which the test agent had been applied was not placed in the glass cylinder in Step 4.

The number of subjects in sensory evaluation was 15. Assuming that no smell is "0," a barely detectable smell is "1," a weak smell that enables a subject to tell what the smell is is "2," an easily detectable smell is "3," a strong smell is "4," and an intense smell is "5," each subject was asked to evaluate the blank and the test agent on a scale of 0 to 5. As a result, the average of 15 subjects was 2.33 for the blank, and 1 for the test agent. According to a deodorant efficacy test method by Air Fresheners and Deodorizers Conference, it can be said that the test agent produces a high deodorizing effect because the intensity of the odor is decreased by one scale or more as compared to that of the blank.

(Deodorizing Effect Confirmation Test for Nonenal)

Next, a deodorizing effect confirmation test for the deodorizing effect of the space deodorant on nonenal as one of various types of malodors in a house will be described. A test system is similar to that in the case of trichloroanisole. A difference is that 0.4 mL of 0.4% nonenal in 99% ethanol was dropped onto the paper filter in Step 2.

A sensory evaluation method was the same as that in the case of nonenal. The results of the sensory evaluation show that the average of 15 subjects was 4.33 for the blank, and 3.2 for the test agent. According to a deodorant efficacy test method by Air Fresheners and Deodorizers Conference, it can be said that the test agent produces a high deodorizing effect because the intensity of the odor is decreased by one scale or more as compared to that of the blank.

(Viral Inactivation Test by Spatial Contact)

As a result of study by those including the inventor of the present application, it was confirmed that benzyl alcohol that is an active component of the indoor space sanitizing agent of the present invention produces a viral inactivation effect in a space. Hereinafter, the viral inactivation effect will be described.

In this test, the indoor space sanitizing agent containing benzyl alcohol as the active component was volatilized into a space, and the effect of inactivating viruses on an object surface in the space was checked.

1. Place a glass cylinder with a volume of 10 L, and place in the glass cylinder a glass petri dish in which an intended amount of benzyl alcohol (the indoor space sanitizing agent) was placed. The amount of benzyl alcohol was 0.002 mg, and after the whole amount of benzyl alcohol was volatilized, the benzyl alcohol concentration in the air in the glass cylinder was 0.05 ppm. When the amount of benzyl alcohol was 0.0009 mg, the benzyl alcohol concentration in the air in the glass cylinder was 0.02 ppm.
2. Place a saturated aqueous solution for humidity control in the glass cylinder.
3. Apply 50 μL of virus to a calico fabric of 2 cm × 2 cm.
4. Place the calico fabric in the glass cylinder, and expose the calico fabric to air containing volatilized benzyl alcohol for 30 minutes or one hour.
5. Take the exposed calico fabric out of the glass cylinder, and put the calico fabric in a microtube.
6. Add 350 μL of a phosphate buffer solution (PBS) to impregnate the calico fabric with PBS, and tap the microtube such that PBS moves up and down.
7. Make a hole at a bottom portion of the microtube containing the calico fabric and PBS by heated tweezers, and place another microtube below the microtube. The two microtubes were stacked on each other and centrifugalized at 25°C and 10000 rpm for one minute in the stacked state.

8. Employ microtubes of the number of samples $\times$ 8, and dispense 450 $\mu$L of DMEM into each microtube.

9. After centrifugation, take 50 $\mu$L of a virus fluid dropped into the lower microtube, and put the virus fluid into one of the microtubes, into which DMEM was dispensed, for dilution. Take 50 $\mu$L of the diluted virus fluid and perform an eight-fold serial dilution in the manner as described above.

10. Employ a culture plate where each culture cell was cultured, and remove a culture solution from each well with an aspirator.

11. Dispense 50 $\mu$L of the virus fluid after the eight-fold serial dilution into each well.

12. Incubate the virus fluid at 37°C for 60 minutes in an incubator with 5% carbon dioxide.

13. After reaction, remove the virus fluid, inject 100 $\mu$L of DMEM into each well, which was cultured for four days.

In this step, in the case of influenza virus, pour a mixture of DMEM and Trypsin into each well to make 100 $\mu$L/well of DMEM + 30 $\mu$g/mL of Trypsin.

14. Pour about 100 $\mu$L of ethanol and acetic acid into each well at a ratio (a weight ratio) of ethanol:acetic acid = 4:1, and immobilize the mixture. Time was about 40 minutes.

15. After removing the immobilized solution, pour 30 $\mu$L of 0.5% amido black in 45% ethanol and 30 $\mu$L of 10% acetic acid, and stain the cells.

16. Immerse the entire plate in a container containing water, and wash away the stain solution so as not to damage the cells.

17. Obtain a virus infectivity titer $TCID_{50}$ by an ordinary method.

The above-described steps (Steps 1 to 17) were performed for influenza virus and feline calicivirus. Blank was also provided. For the blank, no benzyl alcohol was placed in Step 1 above, and no spatial contact between the virus and benzyl alcohol was made in Step 4 above. Moreover, three different test systems with varied benzyl alcohol concentrations, that is, 0.02 ppm, 0.05 ppm, and 0.1 ppm, in the glass cylinder in Step 1 above were provided, and for each of these test systems, the tests using influenza virus and feline calicivirus were performed.

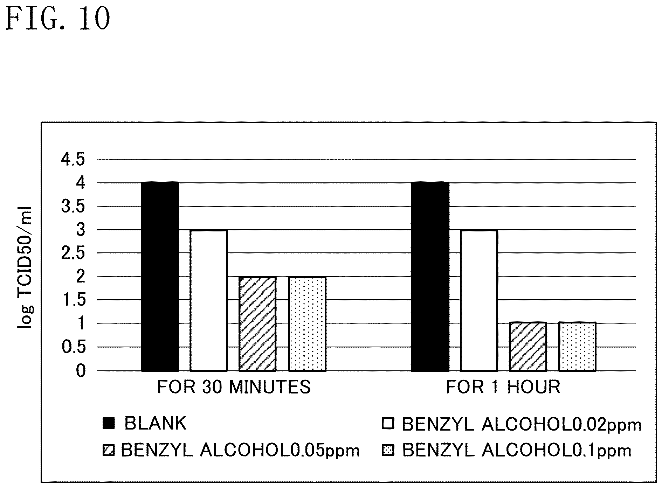

The test results using influenza virus are shown in a graph of FIG. 10. The graph shows cases where spatial contact was made between influenza virus and benzyl alcohol for 30 minutes and for one hour in Step 4 above. The left side of the graph shows the case of spatial contact for 30 minutes and the right side of the graph shows the case of spatial contact for one hour. The effect of inactivating influenza virus is produced even when the concentration of benzyl alcohol is 0.02 ppm, but 99% or more of the influenza virus can be inactivated by spatial contact for one hour when the concentration of benzyl alcohol is 0.05 ppm or more.

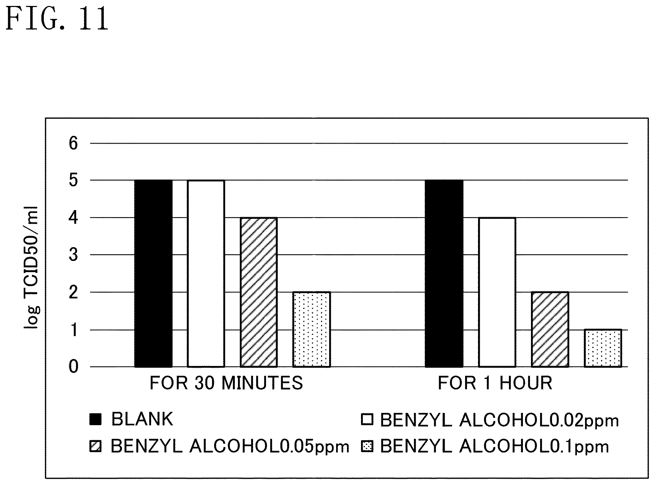

The test results using feline calicivirus are shown in a graph of FIG. 11. The effect of inactivating feline calicivirus is produced by spatial contact for one hour even when the concentration of benzyl alcohol is 0.02 ppm, but 99% or more of feline calicivirus can be inactivated by spatial contact for one hour when the concentration of benzyl alcohol is 0.05 ppm or more.

(Antiviral Effect Duration Test)

At least part of the indoor space sanitizing agent (benzyl alcohol) released to the space adheres to a surface of an object in the space. The tests assumed that after treatment of a space with benzyl alcohol, the benzyl alcohol adhered to a surface of an object in the space, and was performed to check how long the antiviral effect on the surface of the object would last after the adhesion of the benzyl alcohol to the object surface.

1. Apply a designated amount of benzyl alcohol to a calico fabric of 2 cm $\times$ 2 cm, and maintain the calico fabric under static conditions in an open space for the designated number of days (three days, seven days, 14 days, 21 days).

2. Apply 50 $\mu$L of influenza virus or feline calicivirus to the calico fabric after a lapse of the designated number of days for direct contact for two hours (the ISO standards).

3. After contact, place the calico fabric in a microtube.

4. Add 350 $\mu$L of a phosphate buffer solution (PBS) to impregnate the calico fabric with PBS, and tap the microtube such that PBS moves up and down.

5. Make a hole at a bottom portion of the microtube containing the calico fabric and PBS by heated tweezers, and place another microtube below the microtube. The two microtubes were stacked on each other and centrifugalized at 25°C and 10000 rpm for one minute in the stacked state.

6. Employ microtubes of the number of samples $\times$ 8, and dispense 450 $\mu$L of DMEM into each microtube.

7. After centrifugation, take 50 $\mu$L of a virus fluid dropped into the lower microtube, and put the virus fluid into one of the microtubes, into which DMEM was dispensed, for dilution. Take 50 $\mu$L of the diluted virus fluid and perform an eight-fold serial dilution in the manner as described above.

8. Employ a culture plate where culture cells were cultured, and remove a culture solution from each well with an aspirator.

9. Dispense 50 μL of the virus fluid after the eight-fold serial dilution into each well.

10. Incubate the virus fluid at 37°C for 60 minutes in an incubator with 5% carbon dioxide.

11. After reaction, remove the virus fluid, inject 100 μL of DMEM into each well, which was cultured for four days.

In this step, in the case of influenza virus, pour a mixture of DMEM and Trypsin into each well to make 100 μL/well of DMEM + 30 μg/mL of Trypsin.

12. Pour about 100 μL of ethanol and acetic acid into each well at a ratio (a weight ratio) of ethanol:acetic acid = 4:1, and immobilize the mixture. Time was about 40 minutes.

13. After removing the immobilized solution, pour 30 μL of 0.5% amido black in 45% ethanol and 30 μL of 10% acetic acid, and stain the cells.

14. Immerse the entire plate in a container containing water, and wash away the stain solution so as not to damage the cells.

15. Obtain a virus infectivity titer $TCID_{50}$ by an ordinary method.

The above-described steps (Steps 1 to 15) were performed for influenza virus and feline calicivirus. Blank was further provided. For the blank, no benzyl alcohol was applied in Step 1.

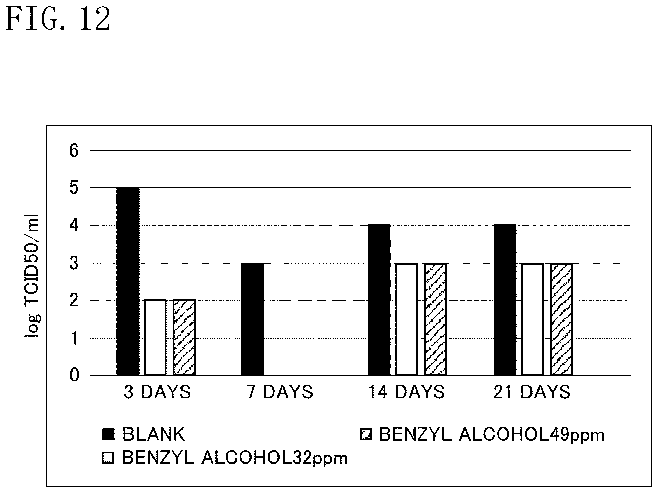

The test results using influenza virus are shown in a graph of FIG. 12. Assuming the cases in which the space was treated with a benzyl alcohol concentration in the air of 32 ppm and the space was treated with a benzyl alcohol concentration in the air of 49 ppm, tests were conducted for each of the cases where the designated number of days was three days, seven days, 14 days, and 21 days. Suppose that the space is treated with a benzyl alcohol concentration in the air of 32 ppm, and that the space is, for example, a room of 3.6 m × 3.6 m, then about 1.4 μg of the benzyl alcohol adheres to a region of 2 cm × 2 cm in the room if it is assumed that the whole amount of the benzyl alcohol adheres to the floor surface after the treatment. Thus, in the test assuming 32 ppm, the designated amount of benzyl alcohol to be applied to the calico fabric in Step 1 was set to be 1.4 μg. Similarly, suppose that the space is treated with a benzyl alcohol concentration in the air of 49 ppm, 2.1 μg of the benzyl alcohol adheres to a region of 2 cm × 2 cm. Thus, in the test assuming 49 ppm, the designated amount of benzyl alcohol to be applied to the calico fabric in Step 1 was set to 2.1 μg.

As shown in FIG. 12, the treatment of the space with a benzyl alcohol concentration in the air of 32 ppm can inactivate 99.9% or more of the influenza virus for seven days after the treatment by the benzyl alcohol adhering to the surface of the object in the space.

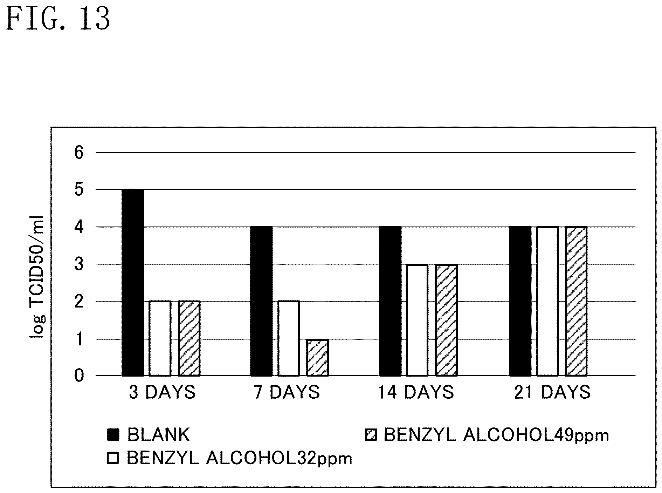

The test results using feline calicivirus are shown in a graph of FIG. 13. As shown in FIG. 13, the treatment of the space with a benzyl alcohol concentration in the air of 49 ppm can inactivate 99% or more of the feline calicivirus for seven days after the treatment by the benzyl alcohol adhering to the surface of the object in the space.

(Floating Virus Inactivation Test)

Next, an inactivation test for virus (floating virus) floating in the air will be described. A test agent in this test is a total release aerosol containing benzyl alcohol. Specifically, the aerosol is stored in an aerosol container as an aerosol agent having a composition as shown in Table 2, and the whole amount of the contents is sprayed by press-down of a spray button. A test space is a chamber assumed to be a room of about two tatami mats, specifically, a size of 186 cm in width, 186 cm in depth, and 186 cm in height. The benzyl alcohol concentration in the air when the whole amount of the test agent was sprayed into the test space was 26 ppm.

1. Take coliphage stored in a deep freezer at -40°C out of the deep freezer, and melt the coliphage at room temperature. In this test, the coliphage was used instead of, e.g., influenza virus. The coliphage is assumed to be a substitute for, e.g., influenza virus because the coliphage has a structure close to that of enveloped viruses.

2. Add 10 μL of coliphage to 10 mL of saline and stir the mixture.

3. Place the phage solution in a nebulizer, and spray the phage solution into the chamber for 15 minutes.

4. Substantially at the same time as the start of spraying the phage solution, start spraying the test agent (until the whole amount was sprayed, which was about several tens of seconds).

5. Collect the floating phage by an impinger when 15 minutes elapsed, when 30 minutes elapsed, when one hour elapsed, when two hours elapsed, when three hours elapsed, and when four hours elapsed from the start of spraying.

6. Add 450 μL of an LB liquid culture medium to a 1.5 mL sterile tube.

7. Add the collected phage solution to the 1.5 mL sterile tube, and perform a serial dilution until $10^{-5}$.

8. Transfer a preculture solution of Escherichia coli for the phage into a 50 mL conical tube, and perform centrifugation at 1500 rpm for 15 minutes.

9. After the centrifugation, fill up to 10 mL.

10. Dispense 350 μL of an Escherichia coli solution into a new 1.5 mL sterile tube.

11. Add 350 μL diluted solution of the phage solution to the Escherichia coli solution dispensed into the tube to make a mixture.

12. Take an LB agar medium (a petri dish or an LB 6-hole plate) out of a refrigerator, and move the LB agar medium into an incubator at 40°C.

13. Add 100 μL of the mixture (in the case of using the 6-hole plate) or 200 μL of the mixture (in the case of using the petri dish) to LB soft agar prepared to be 40°C to 43°C in a temperature controlled bath.

14. Pour the whole amount of the soft agar, to which the mixture was added, to the agar medium, and leave it for a certain period of time to solidification.

15. After the solidification, store the petri dish or the 6-hole plate in the incubator at 40°C, and check plaques after 24 hours.

The test results are shown in Table 1.

[Table 1]

|  | Time | Viable Cell Count (CFU/mL) |
|---|---|---|
| Blank | 15 min. | 4.E+06 |
|  | 30 min. | 5.E+06 |
|  | 1 hour | 6.E+06 |
|  | 2 hours | 3.E+04 |
|  | 3 hours | 9.E+02 |
|  | 4 hours | 7.E+04 |

|  | Time | Viable Cell Count (CFU/mL) | Sanitization Rate (%) |
|---|---|---|---|
| Example | 15 min. | 4.E+04 | 99.15% |
|  | 30 min. | 3 .E+04 | 99.38% |
|  | 1 hour | 3.E+03 | 99.94% |
|  | 2 hours | 0.E+00 | 100.00% |
|  | 3 hours | 0.E+00 | 100.00% |
|  | 4 hours | 0.E+00 | 100.00% |

Blank shows a case of a chamber in which treatment with the test agent was not performed in Step 4. Time in Blank is the time in Step 5 above. In Example, the whole amount of the test agent was sprayed into the chamber immediately after the phage solution was sprayed into the chamber in Step 3 above. The time in Example in Table 1 is time elapsed from the completion of spraying the indoor space sanitizing agent, and is substantially the same as the time in Step 4 above.

[Table 2]

|  | Weight (g) | Conversion Value (ml) |
|---|---|---|
| Benzyl Alcohol | 0.750 |  |
| 99% Synthetic Alcohol | 3.846 | 4.875 |
| LPG4.0 | 7.758 | 11.667 |
| Total | 12.355 | 17.292 |

As shown in Table 1, in Example, 99% or more of the coliphage was inactivated when 15 minutes elapsed from the completion of spraying, and 99.99% or more of the coliphage was inactivated when two hours elapsed from the completion of spraying.

(Efficacy Test for Staphylococcus Aureus)

As a result of study by those including the inventor of the present application, it was found that benzyl alcohol that is an active component of the indoor space sanitizing agent of the present invention produces sanitizing and antibacterial effects. Thus, an efficacy test for Staphylococcus aureus will be next described as the test of the efficacy of the indoor space sanitizing agent. Test bacteria were Staphylococcus aureus NBRC12732: Gram-positive coccus. As a preculture medium, a nutrient liquid culture medium (a common agar medium without agar) was prepared, and about 300 ml of the nutrient liquid culture medium was dispensed into each Sakaguchi flask. A cotton wire was set in the Sakaguchi flask, and was sterilized in an autoclave. The liquid culture medium becomes available when the temperature of the liquid culture medium has decreased to about a body temperature. One inoculation loop of the bacteria in the middle of subculture was taken, and was seeded in the liquid culture medium. The bacteria were shake-cultured for 48 hours in a shaking incubator in a culture room. This resulted in approximately 10^8 CFU/ml. The nutrient liquid culture medium was made to contain, in 1000 ml, 5.0 g of meat extract, 10.0 g of peptone, 5.0 g of sodium chloride, and 1000 g of ion-exchanged water.

In this test, 10 ml of a bacterial suspension, which had been prepared to be 10^7 CFU/ml, was first taken and was placed in a centrifuge. The supernatant besides the resultant precipitated bacteria was discarded, to which a 1 ml of saline was added to prepare a 10^6 CFU/ml bacterial solution. Then, 200 μl of the bacterial solution prepared to be 10^6 CFU/ml was taken, and was applied to a calico fabric of 2 cm × 2 cm. The calico fabric and a test agent in a plastic cup were separately placed in a 10 L cylinder to cause a reaction for eight hours and 24 hours. The test agent was 132 μl of the indoor space sanitizing agent (benzyl alcohol), and evaporated naturally in the 10 L cylinder.

After the reaction, the bacteria were washed out of the calico fabric, and were diluted to 10^-1 to 10^-5 with an SCDLP liquid culture medium. Of such bacteria, 200 μl was seeded in an SCDLP agar medium. The number of colonies was counted after 48 hours.

The test results show that after eight hours, the viable cell count is 590000 CFU/ml in the case of the blank, whereas the viable cell count is 0 CFU/ml in the case of the indoor space sanitizing agent. That is, a sanitization rate is 99.99% or more. After 24 hours, the viable cell count is 105000 CFU/ml in the case of the blank, whereas the viable cell count is 0 CFU/ml in the case of the indoor space sanitizing agent. That is, a sanitization rate is 99.99% or more.

If all the test agent evaporates, the benzyl alcohol concentration in the air in the 10 L cylinder will be 2.9 ppm at the maximum. However, since the amount of actually evaporated benzyl alcohol was about 7.5% of the initial amount, the concentration in the air in the 10 L cylinder was about 0.2 ppm in this test.

(Efficacy Test for Escherichia Coli)

Next, an efficacy test for Escherichia coli will be described as the test for the efficacy of the indoor space sanitizing agent. Test bacteria were Escherichia coli. A preculture medium was the same as that used in the "Test for Staphylococcus Aureus." However, the shake culture time in the shaking incubator was 24 hours. This resulted in approximately 10^8 CFU/ml. In this test, 10 ml of a bacterial suspension, which had been prepared to be 10^7 CFU/ml, was first taken and was placed in a centrifuge. The supernatant besides the resultant precipitated bacteria was discarded, to which a 1 ml of saline was added to prepare a 10^6 CFU/ml bacterial solution. Then, 200 μl of the bacterial solution prepared to be 10^6 CFU/ml was taken, and was applied to a calico fabric of 2 cm × 2 cm. The calico fabric and a test agent in a plastic cup were separately placed in a 10 L cylinder to cause a reaction for eight hours and 24 hours. The test agent was 132 μl of the indoor space sanitizing agent (benzyl alcohol), and evaporated naturally in the 10 L cylinder.

After the reaction, the bacteria were washed out of the calico fabric, and were diluted to 10^-1 to 10^-5 with an SCDLP liquid culture medium. Of such bacteria, 200 μl was seeded in an SCDLP agar medium. The number of colonies was counted after 24 hours.

The test results show that after eight hours, the viable cell count is 6000000 CFU/ml in the case of the blank, whereas the viable cell count is 0 CFU/ml in the case of the indoor space sanitizing agent. That is, a sanitization rate is 99.99% or more. After 24 hours, the viable cell count is 6000000 CFU/ml in the case of the blank, whereas the viable cell count is 0 CFU/ml in the case of the indoor space sanitizing agent. That is, a sanitization rate is 99.99% or more.

If all the test agent evaporates, the benzyl alcohol concentration in the air in the 10 L cylinder will be 2.9 ppm at the maximum. However, since the amount of actually evaporated benzyl alcohol was about 7.5% of the initial amount, the concentration in the air in the 10 L cylinder was about 0.2 ppm in this test.

The results of reactions, under similar test conditions, with the calico fabric for eight hours in each of the following cases will be described, that is, cases of the maximum benzyl alcohol concentration in the air of 2.9 ppm (132 mg of benzyl alcohol as the test agent), the maximum benzyl alcohol concentration in the air of 1 ppm (46.55 mg of benzyl alcohol as the test agent), the maximum benzyl alcohol concentration in the air of 0.1 ppm (4.65 mg of benzyl alcohol as the test agent), the maximum benzyl alcohol concentration in the air of 0.01 ppm (0.47 mg of benzyl alcohol as the test agent), and the maximum benzyl alcohol concentration in the air of 0.001 ppm (0.047 mg benzyl alcohol as the test agent). In any of these concentrations in the air, the viable cell count was 0 CFU/ml, and the sanitization rate was 99.99% or more. That is, a sufficient sanitizing effect can be produced at least with a benzyl alcohol concentration in the air of 0.001 ppm.

(Test Results in Case of Using Indoor Space Sanitizing Device)

Next, an efficacy test for Escherichia coli will be described as the test for the efficacy of the indoor space sanitizing device 1. Test bacteria were Escherichia coli. A preculture medium was the same as that used in the "Test for Staphylococcus Aureus." In this test, 10 ml of a bacterial suspension, which had been prepared to be 10^7 CFU/ml, was first taken and was placed in a centrifuge. The supernatant besides the resultant precipitated bacteria was discarded, to which a 1 ml of saline was added to prepare a 10^6 CFU/ml bacterial solution. Then, 200 $\mu$l of the bacterial solution prepared to be 10^6 CFU/ml was taken, and was applied to a calico fabric of 2 cm $\times$ 2 cm.

The indoor space sanitizing device 1 was maintained under static conditions at the center of the bottom surface of a Peet-Grady chamber (1.82 m in length $\times$ 1.82 m in width $\times$ 1.82 m in height). The liquid agent holding body 4 of the indoor space sanitizing device 1 was impregnated with 2.0 g of benzyl alcohol as the indoor space sanitizing agent.

Calico fabric was placed on the bottom surface of the Peet-Grady chamber at positions 30 cm apart (a first position), 60 cm apart (a second position), 90 cm apart (a third position), and 120 cm apart (a fourth position) from the indoor space sanitizing device 1. Calico fabric was also placed on a side surface of the Peet-Grady chamber at positions 60 cm above (a fifth position), 120 cm above (a sixth position), and 180 cm above (a seventh position) from the bottom surface. In this state, the fan 50 of the indoor space sanitizing device 1 was operated for 24 hours.

Thereafter, the bacteria were washed out of the calico fabric, and were diluted to 10^-1 to 10^-5 in an SCDLP liquid culture medium. Of such bacteria, 200 $\mu$l was seeded in an SCDLP agar medium. The number of colonies was counted after 24 hours. In the case of the blank, the viable cell count was 20025000 CFU/ml. On the other hand, the viable cell count was 0 CFU/ml at the first position, the second position, the third position, and the fourth position. That is, a sanitization rate is 99.99% or more. On the other hand, the viable cell count was 336 CFU/ml at the fifth position and 156 CFU/ml at the sixth position. In either case, the sanitization rate is 99.99% or more.

After the completion of the test, the benzyl alcohol concentration in the air in the Peet-Grady chamber was calculated based on the amount of evaporated benzyl alcohol, and it was 0.011 ppm.

As for Staphylococcus aureus, it is difficult to perform a test in the space as mentioned above (the Peet-Grady chamber) because Staphylococcus aureus needs to be handled in Biosafety Level 2 according to the guideline of World Health Organization (WHO). However, as described above in the "Test for Staphylococcus Aureus," evaporation of the benzyl alcohol produces a favorable sanitizing effect on Staphylococcus aureus, as well as on Escherichia coli. It can thus be said that the evaporation of the benzyl alcohol using the indoor space sanitizing device 1 produces a sufficient sanitizing effect on Staphylococcus aureus, as well, in the space as mentioned above (the Peet-Grady chamber), too.

(Efficacy Test for Escherichia Coli)

Next, the results of an efficacy test for Escherichia coli in an open system will be described. First, calico fabrics of 2 cm $\times$ 2 cm were employed as test pieces, and were sterilized in an autoclave. Thereafter, a benzyl alcohol solution (132 $\mu$l) was evaporated naturally to adhere to the calico fabrics. The calico fabrics were maintained under static conditions in an open system for three days, seven days, 10 days, 14 days, 23 days, and 36 days. After 100 $\mu$l of 1.0 $\times$ 10^6 CFU/ml of Escherichia coli was applied to each of the test pieces maintained under static conditions for the designated number of days, the Escherichia coli was cultured for 24 hours, and was washed out with an SCDLP liquid culture medium. After a five-fold serial dilution, Escherichia coli was applied to an SCDLP agar medium, and was cultured for 24 hours. Thereafter, the number of colonies was counted.

As a result, the sanitization rate of the calico fabric maintained under static conditions for three days and seven days after adherence of the benzyl alcohol as the test agent in the open system was 99.99% or more. That is, the antibacterial effect of the benzyl alcohol in the open system was maintained for at least seven days.

Next, the results of a similar test conducted in a closed system will be described. A 50% benzyl alcohol solution (260 $\mu$l) was evaporated naturally to adhere to calico fabrics sterilized in the same manner as described above. The calico fabrics were maintained under static conditions in the closed system (in a Tupperware® closed with a lid) for three days, seven days, 10 days, 14 days, 23 days, and 36 days. After 100 $\mu$l of 1.0 $\times$ 10^6 CFU/ml of Escherichia coli was applied to each of the test pieces maintained under static conditions for the designated number of days, the Escherichia coli was cultured for 12 hours, and was washed out with an SCDLP liquid culture medium. After a five-fold serial dilution, Escherichia coli was applied to an SCDLP agar medium, and was cultured for 24 hours. Thereafter, the number of colonies was counted.

As a result, the sanitization rate of the calico fabric maintained under static conditions for three days, seven days, 10 days, 14 days, 23 days, and 36 days after adherence of the benzyl alcohol in the closed system was 99.99% or more. That is, the antibacterial effect of the benzyl alcohol in the closed system was maintained for at least 36 days.

(Antifungal Test)

Next, an antifungal test (a test for fungi) will be described. Test bacteria were Trichophyton.

First, a spore suspension was made. For a start, 10 ml of 0.05% polysorbate 80-saline was added to a tube of mold slant. After vigorously mixed, spores were scraped off from the slant with, e.g., a medicine spoon so as to be dispersed sufficiently. The spore suspension was filtered using a sterile gauze (75 mm $\times$ 75 mm), and as necessary, the filtered solution was diluted with 0.05% polysorbate 80-saline so as to have 10^7 spores/ml. A single-spore suspension was made in this manner. This process was repeated until a necessary liquid amount is obtained.

Next, a contact method will be described. A 10 L cylinder was employed, and a 100 $\mu$l of a prepared bacterial suspension was applied to a calico fabric of 2 cm $\times$ 2 cm. A petri dish containing the calico fabric and a 260 mg of a 50% benzyl alcohol solution were placed in the 10 L cylinder, and were brought into spatial contact for 24 hours. The test result was that almost no mold was found in the petri dish. In the case of the blank in which ion-exchanged water was used, mold was found in a substantially half region of the petri dish.

As described above, according to the present embodiment, benzyl alcohol is contained as the active component, which makes it possible to obtain high safety and high sanitizing effect on Escherichia coli and Staphylococcus aureus in a stable manner. In addition, an antibacterial and antifungal effect can be also produced.

Release of benzyl alcohol as the deodorizing active component into a space can eliminate malodors such as ammonia, isovaleric acid, diacetyl, trimethylamine, mercaptan, trichloroanisole, and nonenal. In addition, since benzyl alcohol is contained as the active component, not only stability but also high safety can be ensured.

Release of benzyl alcohol as the active component for virus inactivation into a space can inactivate not only viruses floating in the air but also viruses adhering to an object.

The above-described embodiment is merely illustrative in all respects and should not be interpreted in a limited manner. Further, all modifications and changes which come within the meaning and scope of equivalency of the claims are intended to be embraced in the scope of the present invention.

INDUSTRIAL APPLICABILITY

As described above, the space deodorant and the space deodorizing method according to the present invention can be used in, e.g., a house, an office, and a store.

DESCRIPTION OF REFERENCE CHARACTERS

1       Indoor Space Sanitizing Device
2       Cartridge
4       Liquid Holding Body (Impregnated Body)
50      Fan

**Claims**

**1.** An indoor space sanitizing device comprising:

an indoor space sanitizing agent containing benzyl alcohol as an active component;
a holding body having air permeability and configured to hold the indoor space sanitizing agent, wherein the indoor space sanitizing agent held by the holding body is spread by natural convection of air, and
a cartridge including the holding body.

**2.** The indoor space sanitizing device of claim 1, wherein the cartridge includes a case housing the holding body.

**Patentansprüche**

**1.** Desinfektionsvorrichtung für Innenräume, umfassend:

ein Desinfektionsmittel für Innenräume, das Benzylalkohol als Wirkstoff enthält;
einen Haltekörper mit Luftdurchlässigkeit, der so eingerichtet ist, dass er das Desinfektionsmittel für Innenräume aufnimmt, wobei das von dem Haltekörper gehaltene Desinfektionsmittel für Innenräume durch natürliche Luftkonvektion verteilt wird, und
eine Kartusche, die den Haltekörper enthält.

2. Desinfektionsvorrichtung für Innenräume nach Anspruch 1, wobei die Kartusche ein Gehäuse enthält, das den Haltekörper aufnimmt.

**Revendications**

1. Dispositif de désinfection d'espace intérieur comprenant :

   un agent de désinfection d'espace intérieur contenant de l'alcool benzylique comme composant actif ;
   un corps de retenue présentant une perméabilité à l'air et configuré pour retenir l'agent de désinfection d'espace intérieur, dans lequel l'agent de désinfection d'espace intérieur retenu par le corps de retenue est diffusé par convection naturelle de l'air, et
   une cartouche comprenant le corps de retenue.

2. Dispositif de désinfection d'espace intérieur de la revendication 1, dans lequel la cartouche comprend un boîtier renfermant le corps de retenue.

FIG. 1

40a

BACK SIDE

LEFT SIDE

40a

1

2

40

30

5

40a

RIGHT SIDE

FRONT SIDE

EP 4 079 157 B1

FIG. 2

EP 4 079 157 B1

FIG. 3

BACK SIDE

RIGHT SIDE

3a

3a

2

4

3a

3a

3

LEFT SIDE

FRONT SIDE

FIG. 4

FIG. 5

TIME ELAPSED

FIG. 6

FIG. 7

FIG. 8

FIG. 9

FIG. 10

FIG. 11

# FIG. 12

FIG. 13

**EP 4 079 157 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014014589 A
- JP 2001072503 A
- EP 1183053 A
- JP 2001072503 B